**Europäisches Patentamt**

**European Patent Office**

**Office européen des brevets**

(19)

(11) Veröffentlichungsnummer: **0 111 137**
**A2**

(12) # EUROPÄISCHE PATENTANMELDUNG

(21) Anmeldenummer: **83110780.0**

(22) Anmeldetag: **28.10.83**

(51) Int. Cl.³: **A 61 K 9/02**
**A 61 K 31/405**

---

(30) Priorität: **09.11.82 DE 3241263**

(43) Veröffentlichungstag der Anmeldung:
**20.06.84 Patentblatt 84/25**

(84) Benannte Vertragsstaaten:
**AT BE CH DE FR GB IT LI NL SE**

(71) Anmelder: **Troponwerke GmbH & Co. KG**
**Berliner Strasse 156**
**D-5000 Köln 80(DE)**

(72) Erfinder: **Dell, Hans-Dieter, Dr.**
**Kalmüntener Strasse 5**
**D-5060 Bergisch-Gladbach 2(DE)**

(72) Erfinder: **Kraus, Reinhold**
**Pfaffendorfstrasse 77**
**D-5000 Köln 91(DE)**

(72) Erfinder: **Schierstedt, Detlef, Dr.**
**Henri-Dumont-Strasse 2**
**D-5205 St. Augustin 3(DE)**

(74) Vertreter: **Jesse, Ralf-Rüdiger, Dr. et al,**
**Bayer AG Zentralbereich Patente Marken und Lizenzen**
**D-5090 Leverkusen 1 Bayerwerk(DE)**

---

(54) **Indometacin enthaltende rektale Zubereitungen.**

(57) Die vorliegende Erfindung betrifft neue rektale Zubereitungen die Indometacin, Polyethylenglykol und gegebenenfalls ein Formentrennmittel, jedoch keine Kernbildner enthalten, sowie Verfahren zur Herstellung dieser Zubereitungen. Die Herstellung erfolgt:

a) Durch Verpressen der feinteiligen gegebenenfalls granulierten Bestandteile bei vorzugsweise niedrigen Temperaturen zu Rektaltabletten;

b) durch Abfüllen der feinteiligen gegebenenfalls granulierten Bestandteile bei niedrigen Temperaturen in Hartgelatinekapseln, die anschließend mit Polyethylenglykol überzogen werden.

**0111137**

TROPONWERKE GmbH & Co. KG          5000 Köln 80

Je/bo/c

## Indometacin enthaltende rektale Zubereitungen

Die vorliegende Erfindung betrifft neue rektale Zubereitungen die Indometacin, Polyethylenglykol und gegebenenfalls ein Formentrennmittel, jedoch keine Kernbildner enthalten, sowie Verfahren zur Herstellung dieser Zubereitungen.

Die Herstellung erfolgt:

a)   Durch Verpressen der feinteiligen, gegebenenfalls granulierten Bestandteile bei vorzugsweise niedrigen Temperaturen zu Rektaltabletten.

b)   Durch Abfüllen der feinteiligen gegebenenfalls granulierten Bestandteile bei niedrigen Temperaturen in Hartgelatinekapseln die anschließend mit Polyethylenglykol überzogen werden.

Indometacin ist ein Arzneimittel mit entzündungshemmenden, analgetischen und antipyretischen Eigenschaften, das entsprechend der US-PS 3 161 654 hergestellt werden kann. Die chemische Bezeichnung für Indometacin lautet: 5-Methoxy-2-methyl-1-(4-chlor-benzoyl)-indolyl-(3)-essigsäure. Es ist bereits bekannt, Indometacin-Suppositorien unter Verwendung einer Polyethylenglykol-Grundlage herzustellen, vgl. z.B. DE-OS 2 049 112, DE-OS 2 248 777 und DE-OS 3 040 208. Nach der Lehre dieser Schriften ist es jedoch unbedingt notwendig, Kernbildner, z.B. Kochsalz oder Glycerin, zu verwenden.

TP 46-Ausland

Überraschenderweise wurde nun gefunden, daß es möglich ist, rektale Indometacin-Zubereitungen bereitzustellen, die keine Kernbildner und kein Glycerin enthalten und trotzdem eine ausreichende Beständigkeit aufweisen.

Gegenstand der vorliegenden Erfindung sind daher Zubereitungen enthaltend Indometacin, Polyethylenglykol und gegebenenfalls ein Formentrennmittel.

Ein weiterer Gegenstand der vorliegenden Erfindung sind zwei Verfahren zur Herstellung der erfindungsgemäßen Rektal-Zubereitungen, bei denen

a) Polyethylenglykol, Indometacin und gegebenenfalls ein Formentrennmittel fein verteilt, innig gemischt gegebenenfalls granuliert und die fertige Mischung bei niedrigen Temperaturen, bevorzugt unterhalb 25°C, besonders bevorzugt unterhalb 20°C, ganz besonders bevorzugt unterhalb 15°C zu Rektaltabletten verpreßt wird.

b) Polyethylenglykol, Indometacin und gegebenenfalls ein Formentrennmittel fein verteilt, innig gemischt gegebenenfalls granuliert und die fertige Mischung bei niedrigen Temperaturen, bevorzugt unterhalb 25°C, besonders bevorzugt unterhalb 20°C, ganz besonders bevorzugt unterhalb 15°C in Hartgelatinekapseln abgefüllt wird. Die Kapsel kann anschließend nach bekannten Methoden, vorzugsweise durch Sprühverfahren mit einer Hülle aus reinem Polyethylenglykol oder einer PEG-Mischung versehen werden.

TP 46

Die Abfüllung bzw. Verpressung erfolgt bei einer Temperatur, die für das Gemisch günstig ist, d.h. also z.B. in Abhängigkeit von dem verwendeten Glykol und der Menge des eingesetzten Formentrennmittels. Es ist erfindungsgemäß notwendig, die Verpressung bzw. Abfüllung bei niedrigen Temperaturen vorzunehmen, bevorzugt bei Temperaturen unterhalb 25°C, besonders bevorzugt unterhalb 20°C und ganz besonders bevorzugt unterhalb 15°C. Vorzugsweise wird die Verpressung bzw. Abfüllung zwischen 0°C und 25°C, bevorzugt zwischen 0°C und 20°C, besonders bevorzugt zwischen 5°C und 15°C vorgenommen.

Erfindungsgemäß beträgt der Indometacin-Gehalt der fertigen Mischungen 2 bis 50 Gew.-%, vorzugsweise 2 bis 40 Gew.-%, besonders bevorzugt 2 bis 26 Gew.-%.

Erfindungsgemäß kann jedes Polyethylenglykol bzw. Mischungen von Polyethylenglykolen, die pharmazeutisch geeignet sind, verwendet werden. Das Polyethylenglykol (PEG) bzw. Mischungen der PEG müssen jedoch in Wasser und in den Absonderungen der Schleimhäute löslich sein. Vorzugsweise werden Polyethylenglykole eines durchschnittlichen Molekulargewichts von 200 bis 35.000, besonders bevorzugt von 1.500 bis 6.000 eingesetzt. Die Mischungen werden so zubereitet, daß der Polyethylenglykolanteil an den fertigen Rektal-Zubereitungen 50 bis 98 Gew.-%, vorzugsweise 74 bis 98 Gew.-%, besonders bevorzugt 85 bis 98 Gew.-% beträgt.

TP 46

Die fertige Mischung enthält gegebenenfalls bis zu 20 Gew.-%, bevorzugt bis zu 5 Gew.-%, besonders bevorzugt bis zu 3 Gew.-% Formentrennmittel. Als Formentrennmittel kann eins oder können mehrere aus der Gruppe: Magnesiumstearat, Talkum, silikonisierter Talk, Kalzium-, Aluminiumstearat, Stearinsäure, Palmitinsäure, Stärke, entfettetes Milchpulver, Stearyl-, Zetyl- und Myristylalkohol, höhere Alkohole, höhere Fettsäuren sowie ihre Alkali-, Erdalkali- und Aluminiumsalze, Paraffin, hochdisperses $SiO_2$, hydrierte Fette, Silikonemulsionen, vorzugsweise Magnesiumstearat und Talkum eingesetzt werden. Ganz besonders bevorzugt wird Magnesiumstearat in Mengen bis zu 3 Gew.-% eingesetzt. Diese Formentrennmittel werden lediglich aus technischen Gründen benötigt, um die Tabletten besser aus der Form ablösen bzw. die Kapseln besser abfüllen zu können. Ihnen kommt sonst keinerlei Bedeutung zu. Selbstverständlich müssen die Formentrennmittel pharmazeutisch geeignet, d.h. physiologisch unbedenklich sein.

Glycerin und Kernbildner bzw. Nukleierungsmittel, wie z.B. Alkalihalogenide, etc. werden erfindungsgemäß nicht benötigt. Der Zusatz von Glycerin und Kernbildnern war bei den bisherigen Verfahren, den Gießverfahren, notwendig, damit sich das in der Polyethylenglykol-Grundlage gelöste Indometacin nicht zersetzt. Ferner ist bei den bisher bekannten Verfahren nachteilig, daß durch den Zusatz von Glycerin und osmotisch aktiven Kernbildnern eine Reizung des Rektums nicht auszuschließen ist. Es war deshalb hocherwünscht, Zubereitungen mit größerer

TP 46

Stabilität und geringeren Reizerscheinungen im Rektum herzustellen. Außerdem war es erwünscht Produktionsmethoden
zu finden, nach welchen Tabletten bzw. Kapseln zu
stabileren, wirtschaftlicheren und reizloseren rektalen
Arzneiformen hergestellt werden können. Dies ist nach
den erfindungsgemäß vorgeschlagenen Verfahren der Fall.

Bei den erfindungsgemäßen Verfahren wird Polyethylenglykol, Indometacin und gegebenenfalls ein Formentrennmittel fein verteilt, gut gemischt und gegebenenfalls
granuliert.

Die Verpressung zu Rektaltabletten kann nach dem Prinzip
der herkömmlichen Tablettierung (Exzenter oder Rundläufermaschinen) erfolgen.

Das Abfüllen in Rektalkapseln kann auf herkömmlichen
Kapselmaschinen erfolgen. Anschließend kann die Kapsel
mit einer PEG-Hülle versehen werden.

Die Dosierung der rektalen Zubereitungen richtet sich
nach einer Vielfalt von Faktoren, die bei dem jeweils
gegebenen Anwendungsfall vorliegen.

Üblicherweise werden sowohl die Kapseln als auch die
Tabletten zwischen 30 mg und 120 mg Indometacin enthalten.

Die vorliegende Erfindung soll durch die folgenden Beispiele weiter erläutert werden:

TP 46

Beispiele

Verfahren zur Herstellung von Rektaltabletten (A):

Indometacin, Polyglycol und Magenisiumstearat werden pulverisiert. Dann werden die feinteiligen Bestandteile innig gemischt und bei den angegebenen Temperaturen im angegebenen Gewichtsverhältnis verpreßt.

Verfahren zur Herstellung von Rektalkapseln (B):

Die Herstellung der Mischung erfolgt in dem in der Tabelle angegebenen Temperaturbereich zu den angegebenen Gewichtsverhältnissen analog Variante A. Anschließend werden die Mischungen in Hartgelatinekapseln abgefüllt und dann mit PEG überzogen.

TP 46

A)   Rektaltabletten

| Indometacin | Polyethylenglykole | Mg-stearat | Temperatur |
|---|---|---|---|
| 100 mg | PEG 1500 : 840 mg<br>PEG 3000 : 840 mg | 20 mg | 15°C |
| 50 mg | PEG 1500 : 865 mg<br>PEG 3000 : 865 mg | 15 mg | 15°C |
| 100 mg | PEG 3000 : 1600 mg | 40 mg | 20°C |
| 50 mg | PEG 3000 : 1710 mg | 40 mg | 20°C |
| 100 mg | PEG 2000 : 1680 mg | 20 mg | 10°C |
| 50 mg | PEG 2000 : 1730 mg | 20 mg | 10°C |

B)   Rektalkapseln

| Indometacin | Polyethylenglykole | Mg-stearat | Temperatur | PEG im Überzug |
|---|---|---|---|---|
| 100 mg | PEG 2000 : 450 mg | 5 mg | 10°C | PEG 1500 : 400 mg |
| 50 mg | PEG 2000 : 500 mg | 5 mg | 10°C | PEG 1500 : 400 mg |
| 100 mg | PEG 2000 : 450 mg | 5 mg | 10°C | PEG 1500 : 400 mg |
| 50 mg | PEG 2000 : 500 mg | 5 mg | 10°C | PEG 1500 : 400 mg |
| 100 mg | PEG 3000 : 450 mg | 5 mg | 15°C | PEG 2000 : 400 mg |
| 50 mg | PEG 3000 : 500 mg | 5 mg | 15°C | PEG 2000 : 400 mg |

TP 46

Patentansprüche:

1. Rektale-Zubereitung enthaltend Indometacin, Polyethylenglykol und gegebenenfalls ein Formentrennmittel.

2. Zubereitung nach Anspruch 1, enthaltend
   2 bis 50 Gew.-% Indometacin,
   50 bis 98 Gew.-% Polyethylenglykol und gegebenenfalls
   0 bis 20 Gew.-% Formentrennmittel.

3. Zubereitung nach Anspruch 1 oder 2, enthaltend eines oder ein Gemisch von Polyethylenglykolen eines durchschnittlichen Molekulargewichts von 200 bis 35.000.

4. Zubereitung nach einem oder mehreren der Ansprüche 1 bis 3, dadurch gekennzeichnet, daß feste Formentrennmittel, vorzugsweise Magnesiumstearat und/oder Talkum, eingesetzt werden.

5. Zubereitung nach einem oder mehreren der Ansprüche 1 bis 4, dadurch gekennzeichnet, daß bis zu 5 Gew.-%, bevorzugt bis zu 3 Gew.-% Formentrennmittel eingesetzt werden.

6. Verfahren zur Herstellung einer Rektal-Tabletten-Zubereitung nach einem oder mehreren der Ansprüche 1 bis 5, dadurch gekennzeichnet, daß man Polyethylenglykol, Indometacin und gegebenenfalls Formentrennmittel fein verteilt, innig vermischt, gegebenenfalls granuliert und zu Rektaltabletten verpreßt.

TP 46

7. Verfahren zur Herstellung einer Rektal-Kapsel-
Zubereitung nach einem oder mehreren der Ansprüche 1
bis 5, dadurch gekennzeichnet, daß man Polyethylenglykol, Indometacin und gegebenenfalls Formentrennmittel fein verteilt, innig vermischt, gegebenenfalls granuliert, in Hartgelatinekapseln abfüllt
und anschließend mit einer Polyethylenglykolhülle
versieht.

8. Verfahren nach Anspruch 6, dadurch gekennzeichnet,
daß die Verpressung unterhalb 25°C, vorzugsweise
bei Temperaturen zwischen 0°C und 20°C, vorgenommen
wird.

9. Verfahren nach Anspruch 7, dadurch gekennzeichnet,
daß das Abfüllen in Kapseln unterhalb 25°C vorgenommen wird.

10. Verfahren nach Anspruch 7 oder 9, dadurch gekennzeichnet, daß das Abfüllen in Kapseln bei Temperaturen zwischen 0°C und 20°C vorgenommen wird.

TP 46